# EUROPEAN PATENT SPECIFICATION

(11) **EP 2 180 904 B1**
(45) Date of publication and mention of the grant of the patent: **12.12.2012**
(21) Application number: 08751249.7
(22) Date of filing: 19.05.2008
(51) Int. Cl.: A61L 15/42

(54) **ZONED APPLICATION OF DECOLORIZING COMPOSITION FOR USE IN ABSORBENT ARTICLES**
ZONENWEISE APPLIKATION EINER ENTFÄRBENDEN ZUSAMMENSETZUNG ZUR VERWENDUNG IN SAUGFÄHIGEN ARTIKELN
APPLICATION PAR ZONES D'UNE COMPOSITION DÉCOLORANTE À EMPLOYER DANS DES ARTICLES ABSORBANTS

(30) Priority: 30.08.2007 US 847562
(43) Date of publication of application: 05.05.2010
(73) Proprietor: KIMBERLY-CLARK WORLDWIDE, INC., Neenah, WI 54956 (US)
(72) Inventor: MACDONALD, John, Gavin, Decatur, GA 30033 (US); SMITH, Molly, K., Atlanta, GA 30317 (US)
(74) Representative: Zimmermann & Partner
(86) International application number: PCT/IB2008/051966
(87) International publication number: WO 2009/027856

(56) References cited:
- EP-A- 1 034 804
- WO-A-03/041752
- WO-A-2006/062679
- WO-A-2008/139341

## Description

### Background of the Invention

Feminine care absorbent articles, such as sanitary napkins and pantiliners, typically include an absorbent layer enclosed between a body facing, liquid permeable topsheet and a liquid impermeable backsheet. The topsheet and backsheet may extend laterally beyond the absorbent and be bonded together to form a peripheral seal around the article. The articles are positioned in the crotch portion of an undergarment for absorption of bodily exudates. A concern with conventional feminine care articles is leakage of fluids when using the articles, particularly from around the side edges of the article. Such leakage may lead to embarrassment for the consumer and a general loss of confidence in use of the articles. Various attempts have been made to incorporate structure in the articles to reduce or prevent leakage, including embossed walls or channels, polymeric or other liquid impermeable barrier walls, and the like. However, such attempts have not been completely successful at eliminating the leakage problem. Proposals have also been made to discharge the color of menses and thus the mental impact of a leakage. A need for improvement nevertheless remains.

### Summary of the Invention

In accordance with one embodiment of the present invention, an absorbent article is disclosed that comprises a liquid permeable layer, a generally liquid impermeable layer, and an absorbent layer disposed between the liquid permeable layer and the generally liquid impermeable layer. A decolorizing composition for bodily exudates is disposed on a surface of the liquid permeable layer in a longitudinally extending boundary zone located on at least one side of a longitudinal centerline of the article. At least a portion of the boundary zone overlies a longitudinal periphery portion of the article. An interior zone is defined adjacent to a laterally inboard dimension of the boundary zone, at least a portion of the interior zone being substantially free of the decolorizing composition.

In accordance with one embodiment of the present invention, a feminine care absorbent article is disclosed that comprises a liquid permeable topsheet, a generally liquid impermeable backsheet, and an absorbent layer disposed between the topsheet and the backsheet. A decolorizing composition for bodily exudates is disposed on a surface of the topsheet in longitudinally extending boundary zones located on opposite sides of a longitudinal centerline of the article. At least a portion of the boundary zones overlie a longitudinal periphery portion of the article. An interior zone is defined between laterally inboard dimensions of the boundary zones, at least a portion of the interior zone being substantially free of the decolorizing composition.

Other features and aspects of the present invention are discussed in greater detail below.

### Brief Description of the Drawings

A full and enabling disclosure of the present invention, including the best mode thereof, directed to one of ordinary skill in the art, is set forth more particularly in the remainder of the specification, which makes reference to the appended figure in which:
Fig. 1 is a perspective and partial cut-away of an absorbent article of one embodiment of the present invention;
Fig. 2 is a cross-sectional view of the absorbent article of Fig. 1 taken along the lines indicated in Fig. 1;
Fig. 3 is a cross-sectional view of another embodiment of the present invention;
Fig. 4 is a top view of an alternative embodiment of the present invention; and
Fig. 5 is a top view of still another embodiment of the present invention.

Repeat use of references characters in the present specification and drawing is intended to represent same or analogous features or elements of the invention.

### Detailed Description of Representative Embodiments

### Definitions

As used herein the term "nonwoven fabric or web" refers to a web having a structure of individual fibers or threads which are interlaid, but not in an identifiable manner as in a knitted fabric. Nonwoven fabrics or webs have been formed from many processes such as for example, meltblowing processes, spunbonding processes, bonded carded web processes, etc. The basis weight of nonwoven webs may generally vary, such as from 5 grams per square meter ("gsm") to 150 gsm, in some embodiments from 10 gsm to 125 gsm, and in some embodiments, from 25 gsm to 120 gsm.

As used herein, the term "meltblown web" generally refers to a nonwoven web that is formed by a process in which a molten thermoplastic material is extruded through a plurality of fine, usually circular, die capillaries as molten fibers into converging high velocity gas (e.g. air) streams that attenuate the fibers of molten thermoplastic material to reduce their diameter, which may be to microfiber diameter. Thereafter, the meltblown fibers are carried by the high velocity gas stream and are deposited on a collecting surface to form a web of randomly disbursed meltblown fibers. Such a process is disclosed, for example, in U.S. Patent No. 3,849,241 to Butin, et al. Generally speaking, meltblown fibers may be microfibers that are substantially continuous or discontinuous, generally smaller than 10 microns in diameter, and generally tacky when deposited onto a collecting surface.

As used herein, the term "spunbond web" generally refers to a web containing small diameter substantially continuous fibers. The fibers are formed by extruding a molten thermoplastic material from a plurality of fine, usually circular, capillaries of a spinnerette with the diameter of the extruded fibers then being rapidly reduced as by, for example, eductive drawing and/or other well-known spunbonding mechanisms. The production of spunbond webs is described and illustrated, for example, in U.S. Patent Nos. 4,340,563 to Appel, et al., 3,692,618 to Dorschner, et al., 3,802,817 to Matsuki, et al., 3,338,992 to Kinney, 3,341,394 to Kinney, 3,502,763 to Hartman, 3,502,538 to Levy, 3,542,615 to Dobo, et al., and 5,382,400 to Pike, et al. Spunbond fibers are generally not tacky when they are deposited onto a collecting surface. Spunbond fibers may sometimes have diameters less than about 40 microns, and are often between 5 to 20 microns.

As used herein, the term "coform" generally refers to composite materials comprising a mixture or stabilized matrix of thermoplastic fibers and a second non-thermoplastic material. As an example, coform materials may be made by a process in which at least one meltblown die head is arranged near a chute through which other materials are added to the web while it is forming. Such other materials may include, but are not limited to, fibrous organic materials such as woody or non-woody pulp such as cotton, rayon, recycled paper, pulp fluff and also superabsorbent particles, inorganic and/or organic absorbent materials, treated polymeric staple fibers and so forth. Some examples of such coform materials are disclosed in U.S. Patent Nos. 4,100,324 to Anderson, et al.; 5,284,703 to Everhart, et al.; and 5,350,624 to Georger, et al.

### Detailed Description

Reference now will be made in detail to various embodiments of the invention, one or more examples of which are set forth below. Each example is provided by way of explanation of the invention, not limitation of the invention. In fact, it will be apparent to those skilled in the art that various modifications and variations may be made in the present invention without departing from the scope or spirit of the invention. For instance, features illustrated or described as part of one embodiment, may be used on another embodiment to yield a still further embodiment. Thus, it is intended that the present invention covers such modifications and variations as come within the scope of the appended claims and their equivalents.

Generally speaking, the present invention is directed to an absorbent article that contains a substrate treated with a decolorizing composition that can discharge the color of bodily exudates (e.g., menses, blood, fecal matter, and so forth). More specifically, the absorbent article contains a liquid permeable layer (e.g., topsheet) defining a boundary zone located at or near its periphery that is treated with the decolorizing composition. In this manner, the decolorizing composition may help discharge color at the edges of the article where leakage is most likely to occur. An interior zone of the liquid permeable layer, however, is left substantially untreated with the decolorizing composition. This enables a user to observe and inspect the bodily exudates for infection or other health related conditions, and also allows the decolorizing composition to be applied only to those portions of the layer needed to achieve the desired effect so that the untreated zone can continue to fulfill its other functions, such as absorbing or wicking fluids, etc.

### I. Decolorizing Composition

The decolorizing composition of the present invention contains an oxidizing agent that is capable of oxidizing hemoglobin or other substances responsible for an unwanted color of the bodily exudates. For example, it is believed that the oxidation of hemoglobin may result in the initial formation of methemoglobin (brown color) followed by protein denaturation resulting in a whitening of the brown color to form a yellow color. Suitable oxidizing agents may include, for instance, peroxygen bleaches (e.g., hydrogen peroxide, percarbonates, persulphates, perborates, peroxyacids, alkyl hydroperoxides, peroxides, diacyl peroxides, ozonides, supereoxides, oxo-ozonides, and periodates); hydroperoxides (e.g., tert-butyl hydroperoxide, cumyl hydroperoxide, 2,4,4-trimethylpentyl-2-hydroperoxide, di-isopropylbenzene-monohydroperoxide, tert-amyl hydroperoxide and 2,5-dimethyl-hexane-2,5-dihydroperoxide); peroxides (e.g., lithium peroxide, sodium peroxide, potassium peroxide, ammonium peroxide, calcium peroxide, rubidium peroxide, cesium peroxide, stromtium peroxide, barium peroxide, magnesium peroxide, mercury peroxide, silver peroxide, zirconium peroxide, hafnium peroxide, titanium peroxide, phosphorus peroxide, sulphur peroxide, rhenium peroxide, iron peroxide, cobalt peroxide, and nickel peroxide); perborates (e.g., sodium perborate, potassium perborate, and ammonium perborate); persulphates (e.g., sodium persulphate, potassium dipersulphate, and potassium persulphate); and so forth.

Preferably, the oxidizing agent includes an unsaturated aliphatic acid or an ester thereof having a carbon chain of at least C₈, in some embodiments at least C₁₀, in some embodiments at least C₁₅, and in some embodiments, C₁₈-C₂₆. The aliphatic acid or ester also typically has more than one carbon-carbon double bond, such as at least 2, in some embodiments at least 3, and in some embodiments, at least 4. Without intending to be bound by theory, it is believed that such unsaturated aliphatic acid molecules undergo auto-oxidation as they react with the oxygen from the air. This process involves the oxidative saturation of double bonds and formation of peroxides that can discharge the color of stains. Table 1 lists various suitable unsaturated fatty acids that may be employed arranged in three groups: omega-6, omega-3, and omega-9, wherein the term "omega" signifies where the first double bond in the carbon backbone of the fatty acid occurs. Omega-6 signifies, for instance, that the first double bond occurs at the sixth carbon from the end of the fatty acid (i.e., the omega minus 6 position).

**TABLE 1**

| Omega-6 fatty acids | | |
|---|---|---|
| Common Name | Lipid Name | Chemical Name |
| Linoleic acid | 18:2 (n-6) | 9,12-octadecadienoic acid |
| γ-linolenic acid | 18:3 (n-6) | 6,9,12-octadecatrienoic acid |
| Eicosadienoic acid | 20:2 (n-6) | 11,14-eicosadienoic acid |
| Dihomo-γ-linolenic acid | 20:3 (n-6) | 8,11,14-eicosatrienoic acid |
| Arachidonic acid | 20:4 (n-6) | 5,8,11,14-eicosatetraenoic acid |
| Docosadienoic acid | 22:2 (n-6) | 13,16-docosadienoic acid |
| Adrenic acid | 22:4 (n-6) | 7,10,13,16-docosatetraenoic acid |
| Docosapentaenoic acid | 22:5 (n-6) | 4,7,10,13,16-docosapentaenoic acid]] |
| Calendic acid | 18:3 (n-6) | 8E,10E,12Z-octadecatrienoic acid |

| Omega-3 fatty acids | | |
|---|---|---|
| Common Name | Lipid Name | Chemical Name |
| α-Linolenic acid (ALA) | 18:3 (n-3) | octadeca-9,12,15-trienoic acid |
| Stearidonic acid | 18:4 (n-3) | octadeca-6,9,12,15-tetraenoic acid |
| Eicosatetraenoic acid | 20:4 (n-3) | eicosa-8,11,14,17-tetraenoic acid |
| Eicosapentaenoic acid (EPA) | 20:5 (n-3) | eicosa-5,8,11,14,17-pentaenoic acid |
| Docosapentaenoic acid | 22:5 (n-3) | docosa-7,10,13,16,19-pentaenoic acid |
| Docosahexaenoic acid (DHA) | 22:6 (n-3) | docosa-4,7,10,13,16,19-hexaenoic acid |

| Omega-9 fatty acids | | |
|---|---|---|
| Common Name | Lipid Name | Chemical Name |
| oleic acid | 18:1 (n-9) | 9-octadecenoic acid |
| eicosenoic acid | 20:1 (n-9) | 11-eicosenoic acid |
| mead acid | 20:3 (n-9) | 5,8,11-eicosatrienoic acid |
| erucic acid | 22:1 (n-9) | 13-docosenoic acid |
| nervonic acid | 24:1 (n-9) | 15-tetracosenoic acid |

The omega-3 and -6 fatty acids tend to function best as decolorizers as they generally contain a large numbers of unsaturated bonds. Particularly suitable unsaturated fatty acids are linoleic acid
(CH₃(CH₂)₄CH=CHCH₂CH=CH(CH₂)₇COOH); α-linoleic acid (CH₃CH₂CH=CHCH₂CH=CHCH₂CH=CH(CH₂)₇COOH); arachidonic acid (CH₃(CH₂)₄CH=CHCH₂CH=CHCH₂CH=CHCH₂CH=CH(CH₂)₃COOH); eicosapentaenoic acid (CH₃(CH₂CH=CH)₅(CH₂)₃COOH); docosahexaenoic acid (CH₃(CH₂CH=CH)₆CH₂CH₂COOH); eicosadienoinc acid, eicosatrienoic acid (CH₃(CH₂)₃(CH₂CH=CH)₃(CH₂)₆COOH); etc. Still other suitable fatty acids may be described in U.S. Patent Application Serial No. 11/801,612, filed on May 10, 2007.

Many unsaturated aliphatic acids are also present as a glyceride component of natural-occurring seed oils, such as safflower, grape or pumpkin or soybean, or linseed, or peanut, or poppy, or perilla or a mixture thereof. These fatty acids can be easily and inexpensively extracted from these common seeds. For example, linoleic acid is stored usually in the form of glycerol and found in the seeds of certain plants, such as grapes, flax, safflowers, and peanuts, and fish. The highest levels of linoleic acid are in safflower (carthame) seeds (68-80%), grape seeds (65-73%), and pumpkin seed oil (45-60%). When hydrolyzed into an acid form, it is believed that the active agent in the linoleic acid attacks the conjugated unsaturated bonds in the structure of the colorant molecule. While pure linoleic and linolenic acids can be used to discharge the stain color, the natural seed oils containing esters of these acids can also be used. Table 2, provides a list of some examples of natural seed oils and their linoleic and linolenic acid content, respectively.

**TABLE 2**

| Seed | Linolenic Acid % Total Oil | Linoleic Acid % Total Oil |
|---|---|---|
| Almond | 0 | 17 |
| Avocado | 0 | 10 |
| Beech | 0 | 32 |
| Brazil | 0 | 24 |
| Cashew | 0 | 6 |
| Chia | 30 | 40 |
| Coconut | 0 | 3 |
| Corn | 0 | 59 |
| Cotton | 0 | 50 |
| Evening Primrose | 0 | 81 |
| Filbert | 0 | 16 |
| Flax | 58 | 14 |
| Grape | 0 | 71 |
| Hemp | 20 | 60 |
| Hickory | 0 | 17 |
| Candlenut | 29 | 40 |
| Macadamia | 0 | 10 |
| Neem | 1 | 20 |
| Olive | 0 | 8 |
| Palm kernel | 0 | 2 |
| Peanut | 0 | 29 |
| Pistachio | 0 | 19 |
| Pumpkin | 8 | 50 |
| Rice bran | 1 | 35 |
| Safflower | 3 | 75 |
| Sesame | 0 | 45 |
| Soybean | 7 | 50 |
| Sunflower | 0 | 65 |
| Walnut | 6 | 51 |
| Wheat germ | 5 | 50 |

If desired, multiple oxidizing agents may be employed in the decolorizing composition. For example, an unsaturated aliphatic acid or ester thereof may be used as a primary oxidizing agent in conjunction with a secondary oxidizing agent that enhances the reaction rate of the aliphatic acid against chromophore molecules. Such a secondary oxidizing agent may include, for example, a stabilized peroxide (e.g., urea peroxide). It is believed that the secondary oxidizing agent helps to accelerate the rate at which cyclic peroxide is formed. As more peroxide develops, the secondary oxidizing agent feeds a self-catalyzing reaction, accelerating the reaction rate to discharge colorant stain. The oxidizer breaks down into alkyl radicals that attack colorant molecules.

Regardless of their form, the decolorizing composition typically contains from 0.01 wt.% to 20 wt.%, in some embodiments from 0.1 wt.% to 10 wt.%, and in some embodiments, from 0.5 wt.% to 5 wt.% of oxidizing agents.

Although the oxidizing agents are effective in decolorizing blood stains, it is often desirable to employ a cell lysing agent to disrupt the membrane of an erythrocyte and thereby boost the ability of the oxidizing agent to decolorize a stain. One particularly suitable type of cell lysing agent is a surfactant, such as a nonionic, anionic, cationic, and/or amphoteric surfactant. Suitable nonionic surfactants may include, for instance, alkyl polysaccharides, amine oxides, block copolymers, castor oil ethoxylates, ceto-oleyl alcohol ethoxylates, ceto-stearyl alcohol ethoxylates, decyl alcohol ethoxylates, dinoyl phenol ethoxylates, dodecyl phenol ethoxylates, end-capped ethoxylates, ether amine derivatives, ethoxylated alkanolamides, ethylene glycol esters, fatty acid alkanolamides, fatty alcohol alkoxylates, lauryl alcohol ethoxylates, mono-branched alcohol ethoxylates, natural alcohol ethoxylates, nonyl phenol ethoxylates, octyl phenol ethoxylates, oleyl amine ethoxylates, random copolymer alkoxylates, sorbitan ester ethoxylates, stearic acid ethoxylates, stearyl amine ethoxylates, synthetic alcohol ethoxylates, tallow oil fatty acid ethoxylates, tallow amine ethoxylates, tridecanol ethoxylates, acetylenic diols, polyoxyethylene sorbitols, and mixtures thereof. Various specific examples of suitable nonionic surfactants include, but are not limited to, methyl gluceth-10, PEG-20 methyl glucose distearate, PEG-20 methyl glucose sesquistearate, C₁₁₋₁₅ pareth-20, ceteth-8, ceteth-12, dodoxynol-12, laureth-15, PEG-20 castor oil, polysorbate 20, steareth-20, polyoxyethylene-10 cetyl ether, polyoxyethylene-10 stearyl ether, polyoxyethylene-20 cetyl ether, polyoxyethylene-10 oleyl ether, polyoxyethylene-20 oleyl ether, an ethoxylated nonylphenol, ethoxylated octylphenol, ethoxylated dodecylphenol, or ethoxylated fatty (C₆-C₂₂) alcohol, including 3 to 20 ethylene oxide moieties, polyoxyethylene-20 isohexadecyl ether, polyoxyethylene-23 glycerol laurate, polyoxyethylene-20 glyceryl stearate, PPG-10 methyl glucose ether, PPG-20 methyl glucose ether, polyoxyethylene-20 sorbitan monoesters, polyoxyethylene-80 castor oil, polyoxyethylene-15 tridecyl ether, polyoxyethylene-6 tridecyl ether, laureth-2, laureth-3, laureth-4, PEG-3 castor oil, PEG 600 dioleate, PEG 400 dioleate, and mixtures thereof. Commercially available nonionic surfactants may include the SURFYNOL® range of acetylenic diol surfactants available from Air Products and Chemicals of Allentown, Pennsylvania; the TWEEN® range of polyoxyethylene surfactants available from Fisher Scientific of Pittsburgh, Pennsylvania; and the TRITON@ range of polyoxyethylene surfactants (e.g., TRITON® X-100, polyoxyethylene-10 isooctylcyclohexyl ether) available from Sigma-Aldrich Chemical Co. of St. Louis, Missouri.

Alkyl glycoside nonionic surfactants may also be employed that are generally prepared by reacting a monosaccharide, or a compound hydrolyzable to a monosaccharide, with an alcohol such as a fatty alcohol in an acid medium. For example, U.S. Patent Nos. 5,527,892 and 5,770,543 described alkyl glycosides and/or methods for their preparation. Commercially available examples of suitable alkyl glycosides include Glucopon™ 220, 225, 425, 600 and 625, all of which are available from Cognis Corp. of Cincinnati, Ohio. These products are mixtures of alkyl mono- and oligoglucopyranosides with alkyl groups based on fatty alcohols derived from coconut and/or palm kernel oil. Glucopon™ 220, 225 and 425 are examples of particularly suitable alkyl polyglycosides. Glucopon™ 220 is an alkyl polyglycoside that contains an average of 1.4 glucosyl residues per molecule and a mixture of 8 and 10 carbon alkyl groups (average carbons per alkyl chain-9.1). Glucopon™ 225 is a related alkyl polyglycoside with linear alkyl groups having 8 or 10 carbon atoms (average alkyl chain-9.1 carbon atoms) in the alkyl chain. Glucopon™ 425 includes a mixture of alkyl polyglycosides that individually include an alkyl group with 8, 10, 12, 14 or 16 carbon atoms (average alkyl chain-10.3 carbon atoms). Glucopon™ 600 includes a mixture of alkyl polyglycosides that individually include an alkyl group with 12, 14 or 16 carbon atoms (average alkyl chain 12.8 carbon atoms). Glucopon™ 625 includes a mixture of alkyl polyglycosides that individually include an alkyl group having 12, 14 or 18 carbon atoms (average alkyl chain 12.8 carbon atoms). Still other suitable alkyl glycosides are available from Dow Chemical Co. of Midland, Michigan under the Triton™ designation, e.g., Triton™ CG-110 and BG-10.

Exemplary anionic surfactants include alkyl sulfates, alkyl ether sulfates, alkyl ether sulfonates, sulfate esters of an alkylphenoxy polyoxyethylene ethanol, α-olefin sulfonates, β-alkoxy alkane sulfonates, alkylauryl sulfonates, alkyl monoglyceride sulfates, alkyl monoglyceride sulfonates, alkyl carbonates, alkyl ether carboxylates, fatty acids, sulfosuccinates, sarcosinates, octoxynol or nonoxynol phosphates, taurates, fatty taurides, fatty acid amide polyoxyethylene sulfates, isethionates, or mixtures thereof. Particular examples of anionic surfactants include, but are not limited to, C₈-C₁₈ alkyl sulfates, C₈-C₁₈ fatty acid salts, C₈-C₁₈ alkyl ether sulfates having one or two moles of ethoxylation, C₈-C₁₈ alkamine oxides, C₈-C₁₈ alkoyl sarcosinates, C₈-C₁₈ sulfoacetates, C₈-C₁₈ sulfosuccinates, C₈-C₁₈ alkyl diphenyl oxide disulfonates, C₈-C₁₈ alkyl carbonates, C₈-C₁₈ alpha-olefin sulfonates, methyl ester sulfonates, and blends thereof. The C₈-C₁₈ alkyl group may be straight chain (e.g., lauryl) or branched (e.g., 2-ethylhexyl). The cation of the anionic surfactant may be an alkali metal (e.g., sodium or potassium), ammonium, C₁-C₄ alkylammonium (e.g., mono-, di-, tri-), or C₁-C₃ alkanolammonium (e.g., mono-, di-, tri). More specifically, such anionic surfactants may include, but are not limited to, lauryl sulfates, octyl sulfates, 2-ethylhexyl sulfates, lauramine oxide, decyl sulfates, tridecyl sulfates, cocoates, lauroyl sarcosinates, lauryl sulfosuccinates, linear C₁₀ diphenyl oxide disulfonates, lauryl sulfosuccinates, lauryl ether sulfates (1 and 2 moles ethylene oxide), myristyl sulfates, oleates, stearates, tallates, ricinoleates, cetyl sulfates, and similar surfactants.

Amphoteric surfactants may also be employed, such as derivatives of secondary and tertiary amines having aliphatic radicals that are straight chain or branched, wherein one of the aliphatic substituents contains from about 8 to 18 carbon atoms and at least one of the aliphatic substituents contains an anionic water-solubilizing group, such as a carboxy, sulfonate, or sulfate group. Some examples of amphoteric surfactants include, but are not limited to, sodium 3-(dodecylamino)propionate, sodium 3-(dodecylamino)-propane-1-sulfonate, sodium 2-(dodecylamino)ethyl sulfate, sodium 2-(dimethylamino)octadecanoate, disodium 3-(N-carboxymethyl-dodecylamino)propane-1-sulfonate, disodium octadecyliminodiacetate, sodium 1-carboxymethyl-2-undecylimidazole, and sodium N, N-bis(2-hydroxyethyl)-2-sulfato-3-dodecoxypropylamine. Additional classes of amphoteric surfactants include phosphobetaines and the phosphitaines. For instance, some examples of such amphoteric surfactants include, but are not limited to, sodium coconut N-methyl taurate, sodium oleyl N-methyl taurate, sodium tall oil acid N-methyl taurate, sodium palmitoyl N-methyl taurate, cocodimethylcarboxymethylbetaine, lauryldimethylcarboxymethylbetaine, lauryldimethylcarboxyethylbetaine, cetyldimethylcarboxymethylbetaine, lauryl-bis-(2-hydroxyethyl)carboxymethylbetaine, oleyldimethylgammacarboxypropylbetaine, lauryl-bis-(2-hydroxypropyl)-carboxyethylbetaine, cocoamidodimethylpropylsultaine, stearylamidodimethylpropylsultaine, laurylamido-bis-(2-hydroxyethyl)propylsultaine, di-sodium oleamide PEG-2 sulfosuccinate, TEA oleamido PEG-2 sulfosuccinate, disodium oleamide MEA sulfosuccinate, disodium oleamide MIPA sulfosuccinate, disodium ricinoleamide MEA sulfosuccinate, disodium undecylenamide MEA sulfosuccinate, disodium wheat germamido MEA sulfosuccinate, disodium wheat germamido PEG-2 sulfosuccinate, disodium isostearamideo MEA sulfosuccinate, cocoamphoglycinate, cocoamphocarboxyglycinate, lauroamphoglycinate, lauroamphocarboxyglycinate, capryloamphocarboxyglycinate, cocoamphopropionate, cocoamphocarboxypropionate, lauroamphocarboxypropionate, capryloamphocarboxypropionate, dihydroxyethyl tallow glycinate, cocoamido disodium 3-hydroxypropyl phosphobetaine, lauric myristic amido disodium 3-hydroxypropyl phosphobetaine, lauric myristic amido glyceryl phosphobetaine, lauric myristic amido carboxy disodium 3-hydroxypropyl phosphobetaine, cocoamido propyl monosodium phosphitaine, lauric myristic amido propyl monosodium phosphitaine, and mixtures thereof.

Cationic surfactants may also be employed in the present invention, such as alkyl dimethylamines, alkyl amidopropylamines, alkyl imidazoline derivatives, quaternized amine ethoxylates, quaternary ammonium compounds, etc. Still other suitable cell lysing agents for use herein include biguanide and derivatives thereof, organic sulfur compounds, organic nitrogen compounds, phenyl and phenoxy compounds, phenolic compounds, aldehydes (e.g., glutaraldehyde or formaldehyde), glyoxal, parabens (e.g., ethyl paraben, propyl paraben, or methyl paraben), alcohols, such as aliphatic alcohols having from 1 to 16 carbon atoms, and preferably from 1 to 6 (e.g., methanol, ethanol, propanol, isopropanol, butanol, pentanol, octanol) and aromatic alcohols having from 6 to 30 total carbon atoms (e.g., naphtol), and mixtures thereof.

Typically, the cell lysing agent is present in such an amount that the ratio of the oxidizing agent to the cell lysing agent is from 1:1 up to 30:1, in some embodiments from 5:1 to 25:1, and in some embodiments, from 10:1 or 20:1. For example, the decolorizing composition may contain from 0.001 wt.% to 5 wt.%, in some embodiments from 0.01 wt.% to 2 wt.%, and in some embodiments, from 0.05 wt.% to 1 wt.% of oxidizing agents, by volume of the cell lysing agents.

Besides the ingredients mentioned above, the decolorizing composition may also contain one or more additional ingredients to impart a variety of different benefits to the composition. For example, the decolorizing composition may contain a chelating agent, which is a substance whose molecules can form one or more bonds with a metal ion. In particular, water often contains metal ions, such as calcium ions, that might react with anionic components (e.g., surfactants, acids, etc.) present within the decolorizing composition. Without being limited by theory, it is believed that a chelating agent can form a complex with such metal ions so that the remaining components are capable of fulfilling their desired function. Some examples of chelating agents that may be used in the decolorizing composition of the present invention include, but are not limited to, ethylenediamines, ethylenediaminetetraacetic acids (EDTA) acid and/or salts thereof, citric acids and/or salts thereof, glucuronic acids and/or salts thereof, polyphosphates, organophosphates, dimercaprols, and so forth.

The decolorizing composition may also contain a preservative or preservative system to inhibit the growth of microorganisms over an extended period of time. Suitable preservatives for use in the present compositions may include, for instance, isothiazolinone, benzoic esters (parabens) (e.g., methylparaben, propylparaben, butylparaben, ethylparaben, isopropylparaben, isobutylparaben, benzylparaben, sodium methylparaben, and sodium propylparaben), benzoic acid, propylene glycols, sorbates, urea derivatives (e.g., diazolindinyl urea), and so forth. Other suitable preservatives include those sold by Sutton Labs, such as "Germall 115" (amidazolidinyl urea), "Germall II" (diazolidinyl urea), and "Germall Plus" (diazolidinyl urea and iodopropynyl butylcarbonate). Another suitable preservative is Kathon CG®, which is a mixture of methylchloroisothiazolinone and methylisothiazolinone available from Rohm & Haas; Mackstat H 66 (available from McIntyre Group, Chicago, Illinois). Still another suitable preservative system is a combination of 56% propylene glycol, 30% diazolidinyl urea, 11 % methylparaben, and 3% propylparaben available under the name GERMABEN® II from International Specialty Products of Wayne, New Jersey.

The decolorizing composition may also include various other components as is well known in the art, such as binders, colorants, colorant stabilizers, photoinitiators, binders, solvents, surfactants, humectants, biocides or biostats, electrolytic salts, pH adjusters, etc. For example, various components for use in a decolorizing composition are described in U.S. Patent Nos. 5,681,380 to Nohr, et al. and 6,542,379 to Nohr, et al. Examples of suitable humectants include, for instance, ethylene glycol; diethylene glycol; glycerin; polyethylene glycol 200, 400, and 600; propane 1,3 diol; propylene-glycolmonomethyl ethers, such as Dowanol PM (Gallade Chemical Inc., Santa Ana, California); polyhydric alcohols; or combinations thereof.

To form the decolorizing composition, its components are first typically dissolved or dispersed in a solvent. For example, one or more of the above-mentioned components may be mixed with a solvent, either sequentially or simultaneously, to form a decolorizing composition that may be easily applied to a substrate. Any solvent capable of dispersing or dissolving the components is suitable, for example water; alcohols such as ethanol or methanol; dimethylformamide; dimethyl sulfoxide; hydrocarbons such as pentane, butane, heptane, hexane, toluene and xylene; ethers such as diethyl ether and tetrahydrofuran; ketones and aldehydes such as acetone and methyl ethyl ketone; acids such as acetic acid and formic acid; and halogenated solvents such as dichloromethane and carbon tetrachloride; as well as mixtures thereof. The concentration of solvent in the decolorizing composition is generally high enough to allow easy application, handling, etc. If the amount of solvent is too large, however, the amount of oxidizing agent deposited might be too low to provide the desired decolorization. Although the actual concentration of solvent employed will generally depend on the nature of the decolorizing composition and the substrate to which it is applied, it is nonetheless typically present in an amount from 40 wt.% to 99 wt.%, in some embodiments from 50 wt.% to 95 wt.%, and in some embodiments, from 60 wt.% to 90 wt.% of the decolorizing composition (prior to drying).

### II. Application Technique

A variety of techniques may be used for applying the decolorizing composition composition to a substrate. For instance, the decolorizing composition may be applied using rotogravure or gravure printing, either direct or indirect (offset). Gravure printing encompasses several well-known engraving techniques, such as mechanical engraving, acid-etch engraving, electronic engraving and ceramic laser engraving. Such printing techniques provide excellent control of the composition distribution and transfer rate. Gravure printing may provide, for example, from 10 to 1000 deposits per lineal inch of surface, or from 100 to 1,000,000 deposits per square inch. Each deposit results from an individual cell on a printing roll, so that the density of the deposits corresponds to the density of the cells. A suitable electronic engraved example for a primary delivery zone is about 200 deposits per lineal inch of surface, or about 40,000 deposits per square inch. By providing such a large number of small deposits, the uniformity of the deposit distribution may be enhanced. Also, because of the large number of small deposits applied to the surface of the substrate, the deposits more readily resolidify on the exposed fiber portions. Suitable gravure printing techniques are also described in U.S. Patent No. 6,231,719 to Garvey, et al. Moreover, besides gravure printing, it should be understood that other printing techniques, such as flexographic printing, may also be used to apply the composition.

Still another suitable contact printing technique that may be utilized in the present invention is "screen printing." Screen printing is performed manually or photomechanically. The screens may include a silk or nylon fabric mesh with, for instance, from 40 to 120 openings per lineal centimeter. The screen material is attached to a frame and stretched to provide a smooth surface. The stencil is applied to the bottom side of the screen, i.e., the side in contact with the substrate upon which the composition is to be printed. The decolorizing composition is painted onto the screen, and transferred by rubbing the screen (which is in contact with the substrate) with a squeegee.

Ink-jet printing techniques may also be employed in the present invention. Ink-jet printing is a non-contact printing technique that involves forcing the ink through a tiny nozzle (or a series of nozzles) to form droplets that are directed toward the substrate. Two techniques are generally utilized, i.e., "DOD" (Drop-On-Demand) or "continuous" ink-jet printing. In continuous systems, ink is emitted in a continuous stream under pressure through at least one orifice or nozzle. The stream is perturbed by a pressurization actuator to break the stream into droplets at a fixed distance from the orifice. DOD systems, on the other hand, use a pressurization actuator at each orifice to break the ink into droplets. The pressurization actuator in each system may be a piezoelectric crystal, an acoustic device, a thermal device, etc. The selection of the type of ink-jet system varies on the type of material to be printed from the print head. For example, conductive materials are sometimes required for continuous systems because the droplets are deflected electrostatically.

In addition to the printing techniques mentioned above, any other suitable application technique may be used in the present invention. For example, other suitable printing techniques may include, but not limited to, such as laser printing, thermal ribbon printing, piston printing, spray printing, flexographic printing, etc. Still other suitable application techniques may include bar, roll, knife, curtain, spray, slot-die, dip-coating, drop-coating, extrusion, stencil application, etc. Such techniques are well known to those skilled in the art.

Regardless of the method of application, the substrate may sometimes be dried at a certain temperature to drive any solvent from the decolorizing composition. For example, the substrate may be heated to a temperature of at least about 50°C, in some embodiments at least about 70°C, and in some embodiments, at least about 80°C. By minimizing the amount of solvent in the decolorizing composition, a larger amount of oxidizing agent may be available for contacting bodily exudates, thereby enhancing it ability to decolorize hemoglobin or other substances. It should be understood, however, that relatively small amounts of solvent may still be present. For example, the dried decolorizing composition may contain a solvent in an amount less than about 10% by weight, in some embodiments less than about 5% by weight, and in some embodiments, less than about 1 % by weight.

The relative percentages and add-on level of the composition may vary to achieve the desired level of decolorization. The "add-on level" is determined by subtracting the weight of the untreated substrate from the weight of the treated substrate (after any optional drying steps), dividing this calculated weight by the weight of the untreated substrate, and then multiplying by 100%. One particular benefit of the present invention is that high add-on levels are achievable without a substantial sacrifice other functions of the substrate. In some embodiments, for example, the add-on level of the decolorizing composition is at least about 0.5%, in some embodiments from 1 % to 40%, and in some embodiments, from 2% to 35%.

### III. Absorbent Article

The decolorizing composite of the present invention is employed in the formation of an absorbent article, such as personal care absorbent articles, such as diapers, training pants, absorbent underpants, incontinence articles, feminine hygiene products (e.g., sanitary napkins), swim wear, baby wipes, and so forth; medical absorbent articles, such as garments, fenestration materials, underpads, bedpads, zoneages, absorbent drapes, and medical wipes; food service wipers; clothing articles; and so forth. The substrate may be employed in any part or layer of the absorbent, so long as it is capable of contacting the target bodily exudates (e.g., blood, menses, fecal matter, etc.).

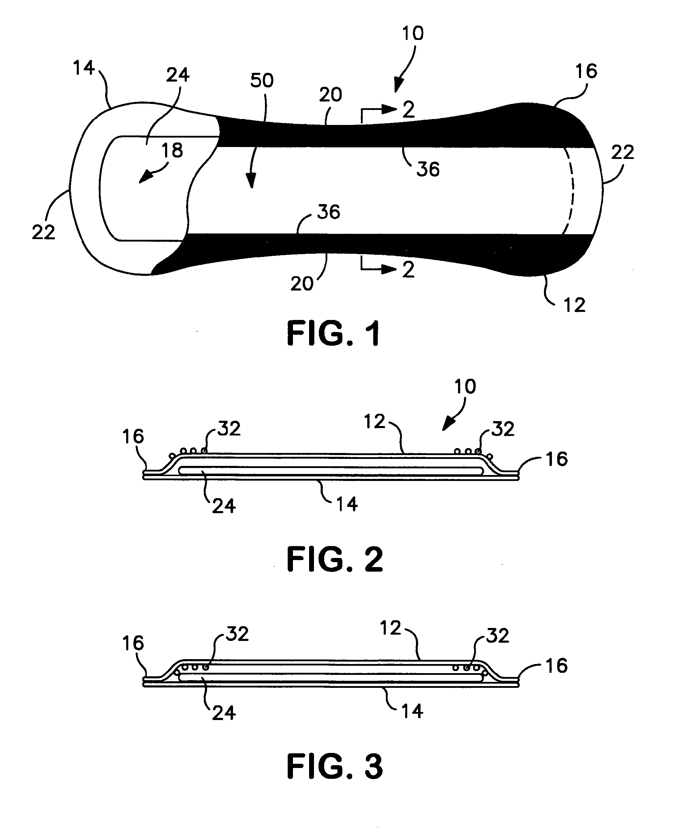

In this regard, various embodiments of an absorbent article that may be formed according to the present invention will now be described in more detail. Referring to Figs. 1 and 2, for example, one embodiment of an absorbent article 10 is shown that includes a generally liquid permeable topsheet 12, a generally liquid impermeable backsheet 14, and an absorbent layer 18 disposed between the topsheet 12 and backsheet 14. The topsheet 12 may surround the absorbent layer 18 so that it completely encases the absorbent article 10. Alternatively, the topsheet 12 and the backsheet 14 may extend beyond the absorbent layer 18 and be peripherally joined together, either entirely or partially, using known techniques. Typically, the topsheet 12 and the backsheet 14 are joined by adhesive bonding, ultrasonic bonding, or any other suitable joining method known in the art, the sealed edges defining an overall sealed peripheral edge 16 of the article 10. The article 10 may take on various geometries but will generally have opposite lateral sides 20 and longitudinal ends 22.

The topsheet 12 is generally designed to contact the body of the user and is liquid-permeable. The liquid permeable topsheet 12 has an outwardly facing surface that may contact the body of the wearer and receive bodily exudates. The topsheet 12 is provided for comfort and conformability and functions to direct bodily exudates away from the body, through the topsheet 12 and toward the absorbent layer 18. The topsheet 12 retains little or no liquid in its structure so that it provides a relatively comfortable and non-irritating surface next to the tissues within the vestibule of a female wearer. The topsheet 12 can be constructed of any woven or nonwoven material which is easily penetrated by bodily exudates which contact the surface of the backsheet. Examples of suitable materials include rayon, bonded carded webs of polyester, polypropylene, polyethylene, nylon, or other heat-bondable fibers, polyolefins, such as copolymers of polypropylene and polyethylene, linear low-density polyethylene, and aliphatic esters such as polylactic acid. Finely perforated film webs and net material can also be used. A specific example of a suitable topsheet material is a bonded carded web made of polypropylene and polyethylene such as that used as topsheet stock for KOTEX® pantiliners and obtainable from Sandler Corporation, Germany. U.S. Patent No. 4,801,494 to Datta, et al. and U.S. Patent No. 4,908,026 to Sukiennik, et al. teach various other topsheet materials that may be used in the present invention.

The topsheet 12 may also contain a plurality of apertures (not shown) formed therethrough to permit body fluid to pass more readily into the absorbent layer 18. The apertures may be randomly or uniformly arranged throughout the topsheet 12, or they may be located only in the narrow longitudinal band or strip arranged along the longitudinal axis X-X of the absorbent article 10. The apertures permit rapid penetration of body fluid down into the absorbent layer 18. The size, shape, diameter and number of apertures may be varied to suit one's particular needs. The topsheet 12 may also be embossed with any desired embossing pattern to define embossed channels. Embossing techniques are well known to those skilled in the art. An embossing pattern not only creates an aesthetically pleasing surface, the channels facilitate intake of menses fluid. Menses will tend to flow along the densified edges of the channels rather than pool on contact points of the topsheet 12.

As stated above, the absorbent article 10 also includes a backsheet 14. The backsheet 14 is generally liquid-impermeable and designed to face the inner surface, i.e., the crotch portion of an undergarment (not shown). The backsheet 14 may permit a passage of air or vapor out of the absorbent article 10, while still blocking the passage of liquids. Any liquid-impermeable material may generally be utilized to form the backsheet 14. For example, one suitable material that may be utilized is a microporous polymeric film, such as polyethylene or polypropylene. In particular embodiments, a polyethylene film is utilized that has a thickness in the range of about 0.2 mils to about 5.0 mils, and particularly between about 0.5 to about 3.0 mils. A specific example of a backsheet material is a polyethylene film such as that used in KOTEX® pantiliners and obtainable from Pliant Corporation, Schaumburg, Illinois, USA.

The absorbent article 10 also contains an absorbent layer 18 positioned between the topsheet 12 and the backsheet 14 that provides capacity to absorb and retain bodily exudates. The absorbent layer 18 may be selected so that it possesses a particular individual total absorbency depending on the intended article of use. For example, for infant care products, the total absorbency can be within the range of about 200-900 grams of 0.9 wt % saline, and can typically be about 500 grams of saline. For adult care products, the total absorbency can be within the range of about 400-2000 grams of saline, and can typically be about 1300 grams of saline. For feminine care products, the total absorbency can be within the range of about 7-50 grams of menstrual fluid, and can typically be within the range of about 30-40 g of menstrual fluid.

The absorbent layer 18 can be any structure or combination of components which are generally compressible, conformable, non-irritating to a wearer's skin, and capable of absorbing and retaining liquids and certain body wastes. For example, the layer 18 may include an absorbent web material 24 of cellulosic fibers (e.g., wood pulp fibers), other natural fibers, synthetic fibers, woven or nonwoven sheets, scrim netting or other stabilizing structures, superabsorbent material, binder materials, surfactants, selected hydrophobic and hydrophilic materials, pigments, lotions, odor control agents or the like, as well as combinations thereof. In a particular embodiment, the absorbent web material is a matrix of cellulosic fluff, and may also include superabsorbent material. The cellulosic fluff may comprise a blend of wood pulp fluff. One preferred type of fluff is identified with the trade designation NB 416, available from Weyerhaeuser Corp., and is a bleached, highly absorbent wood pulp containing primarily soft wood fibers. The absorbent materials may be formed into a web structure by employing various conventional methods and techniques. For example, the absorbent web may be formed with a dry-forming technique, an air forming technique, a wet-forming technique, a foam-forming technique, or the like, as well as combinations thereof. A coform nonwoven material may also be employed. Methods and apparatus for carrying out such techniques are well known in the art.

The absorbent article 10 may also contain additional layers. For example, in one embodiment, the absorbent article 10 may contain a liquid-permeable intake layer (not shown) positioned between the topsheet 12 and a liquid-permeable transfer delay layer (not shown). The liquid-permeable, intake layer may be made of a material that is capable of rapidly transferring, in the z-direction, body fluid that is delivered to the topsheet 12. The intake layer may generally have any shape and/or size desired. In one embodiment, the intake layer has a rectangular shape, with a length equal to or less than the overall length of the absorbent article 10, and a width less than the width of the absorbent article 10. For example, a length of between 150 mm to 300 mm and a width of between 10 mm to 60 mm may be utilized. Any of a variety of different materials are capable of being used for the intake layer to accomplish the above-mentioned functions. The material may be synthetic, cellulosic, or a combination of synthetic and cellulosic materials. For example, airlaid cellulosic tissues may be suitable for use in the intake layer. The airlaid cellulosic tissue may have a basis weight ranging from 10 grams per square meter (gsm) to 300 gsm, and in some embodiments, between 100 gsm to 250 gsm. In one embodiment, the airlaid cellulosic tissue has a basis weight of about 200 gsm. The airlaid tissue may be formed from hardwood and/or softwood fibers. The airlaid tissue has a fine pore structure and provides an excellent wicking capacity, especially for menses.

If desired, a transfer delay layer (not shown) may be positioned vertically below the intake layer. The transfer delay layer may contain a material that is substantially hydrophobic. For example, the transfer delay layer may be a nonwoven fibrous web composed of a relatively hydrophobic material, such as polypropylene, polyethylene, polyester or the like, and also may be composed of a blend of such materials. One example of a material suitable for the transfer delay layer is a spunbond web composed of polypropylene, multi-lobal fibers. Further examples of suitable transfer delay layer materials include spunbond webs composed of polypropylene fibers, which may be round, tri-lobal or poly-lobal in cross-sectional shape and which may be hollow or solid in structure. Typically the webs are bonded, such as by thermal bonding, over about 3% to about 30% of the web area. Other examples of suitable materials that may be used for the transfer delay layer are described in U.S. Patent No. 4,798,603 to Meyer, et al. and U.S. Patent No. 5,248,309 to Serbiak, et al. To adjust the performance of the invention, the transfer delay layer may also be treated with a selected amount of surfactant to increase its initial wettability.

The transfer delay layer may generally have any size, such as a length of about 150 mm to about 300 mm. Typically, the length of the transfer delay layer is approximately equal to the length of the absorbent article 10. The transfer delay layer may also be equal in width to the intake layer, but is typically wider. For example, the width of the transfer delay layer may be from between 50 mm to 75 mm, and particularly about 48 mm. The transfer delay layer typically has a basis weight less than that of the other absorbent members. For example, the basis weight of the transfer delay layer is typically less than about 150 grams per square meter (gsm), and in some embodiments, between 10 gsm to 100 gsm. In one particular embodiment, the transfer delay layer is formed from a spunbonded web having a basis weight of about 30 gsm.

The decolorizing composition of the present invention may generally be applied to any liquid-permeable layer of the absorbent article 10 where it can contact bodily exudates, such as the topsheet 12, intake layer (not shown), transfer delay layer (not shown), and so forth. Regardless of the particular layer to which it is applied, the decolorizing composition is generally present within at least one boundary zone (e.g., two) located at or near the periphery of the layer. The boundary zone generally extends longitudinally, but need extend across the entire length of the layer. The boundary zone may be continuous or discontinuous (e.g., dots, stripes, bands, etc.) and have any desired shape or size. At least one interior zone is positioned adjacent to the boundary zone that constitutes from 5% to 90%, in some embodiments from 10% to 85%, and in some embodiments, from 15% to 75% of the surface area of the layer. The interior zone is left substantially untreated with the decolorizing composition. It should of course be understood that the interior zone may contain a small amount of the composition, so long as it is not present in a high enough amount to decolorize the bodily exudates and prevent a user from inspecting it for infection or other health related conditions.

Referring again to Figs. 1 and 2, for instance, the absorbent article 10 includes two boundary zones 30 of a decolorizing composition 32 defined on the topsheet 12. The zones 30 are defined in a longitudinally extending pattern along opposite lateral sides of a longitudinal centerline of the article 10. At least a portion of each boundary zone 30 has a laterally inboard dimension 36 overlying a longitudinally extending periphery portion of the underlying absorbent layer 18. As illustrated, the boundary zones 30 may extend laterally outward to the sealed peripheral edge 16 of the article 10 to help decolorize bodily exudates at the areas in which they are most likely to leak from the article. A longitudinally extending interior zone 50 is also defined on the topsheet 12 between the opposing boundary zones 30, at least a portion of which is left substantially untreated with the decolorizing composition so that a user is able to monitor the bodily exudates for infection or other health-related conditions.

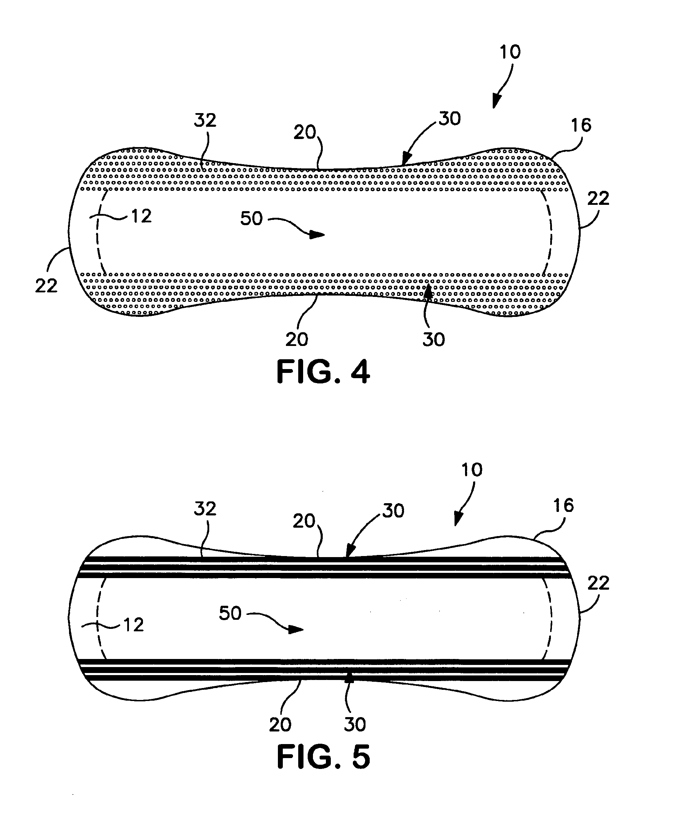

In the embodiment illustrated in Fig. 1, the two longitudinally extending boundary zones 30 are defined by a generally continuous stripe extending longitudinally on opposite sides of a centerline axis of the article 10. Of course, the boundary zones 30 need not be continuous and multiple stripes may be employed. Likewise, the zones 30 also need no extend the entire length of the topsheet 12. Furthermore, any other suitable zone pattern may be employed in the present invention. Fig. 5, for instance, illustrates another type of zone pattern that may be used in the present invention. In this embodiment, sets of multiple closely spaced stripes of the decolorizing composition are spaced apart a certain distance. Each set may contain any number of stripes so long as the desired percentage of uncovered area of liner is maintained between the laterally outboard and laterally inboard sets of stripes. Fig. 4 illustrates an alternative embodiment of a discontinuous pattern for the boundary zones 30. In this embodiment, the boundary zones are defined by individual dot-like portions 32 on the topsheet 12.

The decolorizing composition may be provided on any surface of a substrate to which it is applied. For example, in the embodiments discussed above, the decolorizing composition 32 is provided on a body-facing surface of the topsheet 12 (Fig. 2). In other embodiments, however, the decolorizing composition 32 is provided on an interior surface (facing the absorbent material 24) of the topsheet 12 (Fig. 3).

Although various embodiments of an absorbent article have been described above that may incorporate the benefits of the present invention, it should be understood that other configurations are also included within the scope of the present invention. For instance, other absorbent article configurations are described in U.S. Patent Nos. 5,649,916 to DiPalma, et al.; 6,110,158 to Kielpikowski; 6,663,611 to Blaney, et al.; U.S. Patent Nos. 4,886,512 to Damico et al.; 5,558,659 to Sherrod et al.; 6,888,044 to Fell et al.; and 6,511,465 to Freiburger et al., as well as U.S. Patent Application Publication No. 2004/0060112 A1 to Fell et al.

The present invention may be better understood with reference to the following examples.

### EXAMPLE 1

The ability of a decolorizing composition to discharge the color of blood was demonstrated. Microscope slides were coated with a mixture of linoleic acid, Surfynol™ 465 and water (ratio 1:0.01:1) and spread out by the use of a glass rod. A drop of blood (human) was placed onto each slide and spread out with the glass rod. The control slide was prepared by simply spreading out the drop of blood. The slides were placed in an incubator at 37°C for one hour. The blood color was fully discharged on the experimental slides while no change in color was observed with the control slides.

### EXAMPLE 2

The ability of a decolorizing composition to discharge the color of blood was demonstrated. Four feminine pads were used in a study. Control 1 (C1) just had two drops of human blood placed on the center of the pad. Control 2 (C2) had a light coating of Surfynol™ 465 in water (0.25 ml in 0.5 ml water) placed on the center of the pad followed by two drops of human blood. Experimental pad 1 (E1) had a mixture of linoleic acid and surfactant (0.25 ml surfactant and 0.50 ml of linoleic acid) followed by two drops of human blood. Experimental pad 2 (E2) had a mixture of linoleic acid and surfactant (0.50 ml of surfactant and 0.50 ml of linoleic acid) applied to the center of the pad followed by two drops of human blood. The pads were placed in an incubator at 37°C and observed. Within minutes the red color of the blood on pads E1 and E2 had turned brown and continued to fade with time. At the end of the experiment the blood color on pads E1 and E2 had been fully discharged, leaving only a pale yellow tint. The blood color on the control pads remained essentially unchanged. The blood color on pad C2 was visibly lighter as compared to the color on the C1 pad. This suggests that the surfactant itself had a slight effect on the color, which is thought to result from the ability of the surfactant to lyse the blood cells, allowing the hemoglobin to be slightly oxidized by the air.

### EXAMPLE 3

The ability of a decolorizing composition to discharge the color of blood was demonstrated. Using 4" x 5" sample of cotton, satin, polyester and light-weight Lycra were used in a study where blood (20 µl) or menses (50 µl) was placed onto the center of the fabric samples to mimic a stain situation. To determine potential efficacy of stain removal, 200 µl of a control solution (Surfynol™ 465 surfactant as a 2% wt/wt solution in water) or 200 µl of surfactant solution containing linoleic acid as the stain removal active (1:2 ratio of 2% surfactant solution to linoleic acid) were applied to the area soiled by blood or menses. The samples were then placed in an incubator at 37°C and the color change visually monitored. It is clear that the intense menses color had abated using the identified technology.

### EXAMPLE 4

The ability of a decolorizing composition to discharge the color of blood was demonstrated. In a number of experiments, feminine pads (Kotex® by Kimberly-Clark Corporation) are pretreated with 200 µl of Surfynol™ or Tween™ 20 surfactant in water solution (2% wt./wt.) for use as control samples. Other pads were treated with 200 µl of a mixture of linoleic acid and surfactant (2:1 ratio). All the pads were then insulted with 50 µl of menses. The pads clearly showed that on the control pads, where the menses has wicked to cover a larger patch on the pad, the pad retained the red color. In contrast, the menses on the pad containing the linoleic acid remained fixed in a tight spot and the color has been reduced to a pale yellow.

### EXAMPLE 5

The ability of a decolorizing composition to discharge the color of blood was demonstrated. A series of experiments were conducted to compare the ability of a linoleic acid-based system to commercial spot and stain removers to decolorize blood and menses on textiles. The fabric samples were placed on an aluminum foil sheet. On the center of each sample was placed either 50 µl of menses or 20 µl of blood. Then, 200 µl of either Tide®-to-Go, 2 wt% aqueous Surfynol™ 465 solution, linoleic acid in Surfynol™ 465 solution, Shout® wipe fluid, or Clorox® bleach pen fluid was pipetted onto the sample stain. The spot was gently mixed with the end of a glass rod for 5 seconds. The samples were then placed in an incubator at 37°C and visually observed.

Although both the Tide®-to-go and Shout® stain color were slightly reduced, it appeared to have been due to dilution rather than discharge of the color. The linoleic acid/surfactant system performed the best completely discharging the color of the menses. The Clorox® bleach pen system turned the menses into a black deposit and also bleached the denim. The same results were also found with the blood stain samples. The linoleic acid system performed the best by complete decolorization of the blood spot. The Clorox® bleach pen turned the blood into a black spot coated with a white deposit. When the sample was turned over, the underside of the fabric had a black spot.

### EXAMPLE 6

The ability of a decolorizing composition to discharge the color of blood was demonstrated. The oils containing the highest concentration of linoleic acid are safflower (carthame) seed (68-80%), grape seed (65-73%), and pumpkin seed oil (45-60%). A quick experiment was conducted using a sample of pumpkin seed oil applied to pads. In the study, a feminine pad (e.g., Kotex®) had 300 µl of the pumpkin seed oil placed on it. A second pad had 300 µl of a mixture of pumpkin seed oil and Surfynol™ 465 (ratio 4:1) placed on it. A third pad had 300 µl of the linoleic acid/surfactant mixture used earlier. A fourth pad (control) had 300 µl of water placed on it. The pads were then insulted with 20 µl of human blood. The preliminary results showed that the pumpkin seed oil/surfactant mixture discharged the blood color to almost the same extent as the linoleic acid/surfactant mixture. Although the concentration of linoleic acid in this seed oil product was not known, it was clearly a sufficient amount to reduce blood color. The pumpkin seed oil alone (without surfactant) was not sufficient to affect the color. This study demonstrates that natural oils could be used in the pads, which offer the potential for "natural" product labeling, as well as possible skin health benefit claims. Other oils were also tested on cotton fabric to determine the potential efficacy with blood stains. Safflower and grape seed oils also discharged the red color of blood and significantly reduced the stain color.

### EXAMPLE 7

The ability of a decolorizing composition to discharge the color of blood was demonstrated. Fresh human blood was placed onto the center of cotton squares (3"x 4") and allowed to completely dry by leaving it for 3 hours. The dried stains were blotted with a paper towel to check that they were indeed dried. No blood was observed on the blotting towel and the stain was therefore determined to be completely dry. Onto one stain were placed two drops of the linoleic acid:Surfynol™ 465 mixture (2: 1) and spread over the stain using a glass rod. The samples were then placed in an incubator at 37°C and the color was observed. The stain color was discharged, however it did take twice as long when compared to a fresh blood stain.

### EXAMPLE 8

The ability of a decolorizing composition to discharge the color of blood was demonstrated. About 20 mg of pure bovine hemoglobin was dissolved into 1ml of de-ionized (DI) water. Two drops were placed onto a 3"x 4" square of cotton fabric and the same repeated for the control fabric. Two drops of linoleic acid were then placed onto one of the cotton squares having the hemoglobin "stain." The fabrics were then placed in an incubation chamber (37°C and 50% humidity). The hemoglobin color was discharged within minutes to give a colorless area where the red stain had been. The red color of the hemoglobin was effectively discharged by the linoleic acid.

### EXAMPLE 9

The ability of a decolorizing composition to discharge the color of bilirubin was demonstrated. Bilirubin is a yellow breakdown product of normal heme catabolism in the body. Bilirubin reduction in the gut leads to a product called urobilinogen, which is excreted in the urine. Bilirubin is also further broken down in the intestine by the intestinal microbes via urobilin to a final product called stercobilin which gives feces the characteristic brown color. Bilirubin was dissolved into water (30 mg/ml) and two drops were applied to two separate cotton squares. Then, to one of the orange/yellow stains was applied a mixture of linoleic acid/Surfynol™ 465 (2:1) solution and spread across the stain by use of a glass rod. The samples were then placed into an incubator at 37°C and the color was observed. Within minutes, the linoleic acid treated yellow stain color was discharged to leave a colorless area where the stain had been.

### EXAMPLE 10

The ability of a decolorizing composition to discharge the color of blood was demonstrated. 50 µl of 7% wt/wt hemoglobin in water solution was applied to cotton fabric squares and allowed the hemoglobin to soak into the fabric, creating a brown/red stain. Onto one stain was placed 200 µl of linoleic acid/Surfynol™ 465 mixture (5:1 where Surfynol™ 465 was a 20% wt/wt solution in water) and briefly rubbed with the tip of a glass rod to ensure coverage of the stain. The hemoglobin color was fully discharged in about 2.4 minutes. Onto a second stain was placed 200 µl of a linoleic acid/Surfynol™ 465 (20% Surfynol™ 465 in water)/urea peroxide solution (5:1:0.3) and briefly rubbed with the end of a glass rod to ensure coverage of the stain. The color was completely discharged in 34 seconds. The color discharge role of the small amount of urea peroxide was tested. Thus, 200 µl sample of 0.3%wt/wt urea peroxide in water was applied to an identical hemoglobin stain and rubbed with the tip of a glass rod to ensure coverage of the stain. A slight dilution of the stain color was observed the stain persisted. Even after about 5 hours, the vivid brown/red stain was still clearly visible to the unaided eye.

### EXAMPLE 11

The ability of a decolorizing composition to discharge the color of blood on the boundary zones of a feminine care pad was demonstrated. A solution of linoleic acid and Surfynol™ 465 in water (2% wt/wt linoleic acid in a 20% Surfynol™ 465 water solution in 20 ml) was pipetted only along the edges of a KOTEX® lightdays pad. More specifically, 500 µl was placed along each 4" length of the one side of the pad and then repeated on the other side. The location of the edge of the treatment on the pad was marked with a Sharpie pen. Next 800 µl of menses was placed on the center of the pad. The pad was placed in an incubator (37°C and 90% humidity) and the color observed. When the menses migrated to the edge of the pad, it crossed the pan line and the red color was discharged where it had come in contact with the linoleic acid system.

## Claims

1. An absorbent article, comprising:
a liquid permeable layer;
a generally liquid impermeable layer;
an absorbent layer disposed between the liquid permeable layer and the generally liquid impermeable layer; and
a decolorizing composition for bodily exudates disposed on a surface of the liquid permeable layer in a longitudinally extending boundary zone located on at least one side of a longitudinal centerline of the article, at least a portion of the boundary zone overlying a longitudinal periphery portion of the article, wherein an interior zone is defined adjacent to a laterally inboard dimension of the boundary zone, at least a portion of the interior zone being substantially free of the decolorizing composition;
wherein the decolorizing composition comprises an oxidizing agent, which oxidizing agent is an unsaturated aliphatic acid or ester thereof having a carbon chain of at least C₈ and more than one carbon-carbon double bond.

2. The absorbent article of claim 1, wherein the article comprises a feminine care absorbent article.

3. The absorbent article of claim 2, wherein the article is a sanitary napkin.

4. The absorbent article of any of the foregoing claims, wherein the interior zone constitutes from 5% to 90% of the surface area of the liquid permeable layer.

5. The absorbent article of claim 4, wherein the interior zone constitutes from 15% to 75% of the surface area of the liquid permeable layer

6. The absorbent article of any of the foregoing claims, wherein the boundary zone comprises a laterally outboard dimension generally coextensive with at least a portion of the periphery of the absorbent article.

7. The absorbent article of any of the foregoing claims, wherein the decolorizing composition is disposed in longitudinally extending boundary zones on opposite sides of the longitudinal centerline of the article, at least a portion of the zones overlying longitudinal periphery portions of the absorbent layer, wherein the interior zone is defined between laterally inboard dimensions of the boundary zones.

8. The absorbent article of claim 7, wherein the boundary zones are defined around less than all of a periphery of the article.

9. The absorbent article of claim 7, wherein the boundary zones are defined around an entirety of a periphery of the article.

10. The absorbent article of any of the foregoing claims, wherein the boundary zone comprises a discontinuous pattern of the decolorizing composition.

11. The absorbent article of any of the foregoing claims, wherein the oxidizing agent includes an omega-3 fatty acid, omega-6 fatty acid, an ester thereof, or a mixture thereof.

12. The absorbent article of any of the foregoing claims, wherein the oxidizing agent includes linoleic acid, α-linolenic acid, eicosadienoic acid, eicosatrienoic acid, arachidonic acid, eicosapentaenoic acid, docosahexaenoic acid, an ester thereof, or a mixture thereof.

13. The absorbent article of any of the foregoing claims, wherein the decolorizing composition further comprises a cell lysing agent.

14. The absorbent article of any of claims 1 to 13, wherein the liquid permeable layer is a topsheet of the article.

15. The absorbent article of any of claims 1 to 13, wherein liquid permeable layer is an intake layer, transfer delay layer, or a combination thereof.

## Patentansprüche

1. Absorptionsfähiger Gegenstand, umfassend:
eine flüssigkeitsdurchlässige Lage,
eine im Allgemeinen flüssigkeitsundurchlässige Lage,
eine zwischen der flüssigkeitsdurchlässigen Lage und der im Allgemeinen flüssigkeitsundurchlässigen Lage angeordnete absorptionsfähige Lage; und
eine entfärbende Zusammensetzung für Körperausscheidungen, welche auf einer Oberfläche der flüssigkeitsdurchlässigen Lage angeordnet ist, in einer sich in Längsrichtung erstreckenden Begrenzungszone, welche auf wenigstens einer Seite einer Mittenlinie in Längsrichtung des Gegenstands gelegen ist, wobei wenigstens ein Bereich der Begrenzungszone einen in Längsrichtung peripheren Bereich des Gegenstands überlagert, wobei angrenzend an eine seitlich innere Ausdehnung der Begrenzungszone eine Innenzone definiert ist, wenigstens ein Bereich der Innenzone im Wesentlichen frei von der entfärbenden Zusammensetzung ist;
wobei die entfärbende Zusammensetzung ein Oxidationsmittel enthält, das Oxidationsmittel eine ungesättigte aliphatische Säure oder ein Ester davon mit einer Kohlenstoffkette von wenigstens C₈ und mehr als einer Kohlenstoff-Kohlenstoff-Doppelbindung ist.

2. Absorptionsfähiger Gegenstand nach Anspruch 1, wobei der Gegenstand einen absorptionsfähigen Hygienegegenstand für Frauen umfasst.

3. Absorptionsfähiger Gegenstand nach Anspruch 2, wobei der Gegenstand eine Damenbinde ist.

4. Absorptionsfähiger Gegenstand nach einem der vorhergehenden Ansprüche, wobei die Innenzone von 5% bis 90% der Oberfläche der flüssigkeitsdurchlässigen Lage ausmacht.

5. Absorptionsfähiger Gegenstand nach Anspruch 4, wobei die Innenzone von 15% bis 75% der Oberfläche der flüssigkeitsdurchlässigen Lage ausmacht.

6. Absorptionsfähiger Gegenstand nach einem der vorhergehenden Ansprüche, wobei die Begrenzungszone eine seitlich äußere Ausdehnung umfasst, welche im Allgemeinen mit wenigstens einem Bereich der Peripherie des absorptionsfähigen Gegenstands koextensiv ist.

7. Absorptionsfähiger Gegenstand nach einem der vorhergehenden Ansprüche, wobei die entfärbende Zusammensetzung in sich in Längsrichtung erstreckenden Begrenzungszonen auf gegenüberliegenden Seiten der Mittenlinie in Längsrichtung des Gegenstands angeordnet ist, wobei wenigstens ein Bereich der Zonen periphere Bereiche in Längsrichtung der absorptionsfähigen Lage überlagert, wobei die Innenzone zwischen seitlich inneren Ausdehnungen der Begrenzungszonen definiert ist.

8. Absorptionsfähiger Gegenstand nach Anspruch 7, wobei die Begrenzungszonen um weniger als eine Gesamtheit einer Peripherie des Gegenstands definiert sind.

9. Absorptionsfähiger Gegenstand nach Anspruch 7, wobei die Begrenzungszonen um eine Gesamtheit einer Peripherie des Gegenstands definiert sind.

10. Absorptionsfähiger Gegenstand nach einem der vorhergehenden Ansprüche, wobei die Begrenzungszone ein diskontinuierliches Muster der entfärbenden Zusammensetzung umfasst.

11. Absorptionsfähiger Gegenstand nach einem der vorhergehenden Ansprüche, wobei das Oxidationsmittel eine Omega-3-Fettsäure, Omega-6-Fettsäure, einen Ester davon, oder ein Gemisch davon umfasst.

12. Absorptionsfähiger Gegenstand nach einem der vorhergehenden Ansprüche, wobei das Oxidationsmittel Linolsäure, α-Linolensäure, Eicosadiensäure, Eicosatriensäure, Arachidonsäure, Eicosapentaensäure, Docosahexaensäure, einen Ester davon, oder ein Gemisch davon umfasst.

13. Absorptionsfähiger Gegenstand nach einem der vorhergehenden Ansprüche, wobei die entfärbende Zusammensetzung des Weiteren ein Zell-lysierendes Agens umfasst.

14. Absorptionsfähiger Gegenstand nach einem der Ansprüche 1 bis 13, wobei die flüssigkeitsdurchlässige Lage eine Decklage des Gegenstands ist.

15. Absorptionsfähiger Gegenstand nach einem der Ansprüche 1 bis 13, wobei die flüssigkeitsdurchlässige Lage eine Aufnahmelage, eine Transferverzögerungslage, oder eine Kombination davon ist.

## Revendications

1. Article absorbant, comprenant :
une couche perméable aux liquides ;
une couche généralement imperméable aux liquides ;
une couche absorbante disposée entre la couche perméable aux liquides et la couche généralement imperméable aux liquides ; et
une composition décolorante pour des exsudats corporels disposée sur une surface de la couche perméable aux liquides dans une zone de délimitation s'étendant longitudinalement, située sur au moins un côté d'un axe longitudinal de l'article, au moins une partie de la zone de délimitation recouvrant une partie périphérique longitudinal de l'article, dans lequel une zone intérieure est définie au voisinage d'une dimension latéralement à l'intérieur de la zone de délimitation, au moins une partie de la zone intérieure étant sensiblement exempte de la composition décolorante ;
dans lequel la composition décolorante comprend un agent oxydant, lequel agent oxydant est un acide aliphatique insaturé ou un ester de celui-ci ayant une chaîne carbonée d'au moins C₈ et plus d'une double liaison carbone-carbone.

2. Article absorbant selon la revendication 1, dans lequel l'article comprend un article absorbant de soin féminin.

3. Article absorbant selon la revendication 2, dans lequel l'article est une serviette hygiénique.

4. Article absorbant selon l'une quelconque des revendications précédentes, dans lequel la zone intérieure constitue de 5 % à 90 % de l'aire de surface de la couche perméable aux liquides.

5. Article absorbant selon la revendication 4, dans lequel la zone intérieure constitue de 15 % à 75 % de l'aire de surface de la couche perméable aux liquides.

6. Article absorbant selon l'une quelconque des revendications précédentes, dans lequel la zone de délimitation comprend une dimension latéralement à l'extérieur s'étendant généralement conjointement avec au moins une partie de la périphérie de l'article absorbant.

7. Article absorbant selon l'une quelconque des revendications précédentes, dans lequel la composition décolorante est disposée dans des zones de délimitation s'étendant longitudinalement sur des côtés opposés de l'axe longitudinal de l'article, au moins une partie des zones recouvrant des parties périphériques longitudinales de la couche absorbante, dans lequel la zone intérieure est définie entre des dimensions latéralement à l'intérieur des zones de délimitation.

8. Article absorbant selon la revendication 7, dans lequel les zones de délimitation sont définies autour de moins de toute une périphérie de l'article.

9. Article absorbant selon la revendication 7, dans lequel les zones de délimitation sont définies autour d'une totalité d'une périphérie de l'article.

10. Article absorbant selon l'une quelconque des revendications précédentes, dans lequel la zone de délimitation comprend un motif discontinu de la composition décolorante.

11. Article absorbant selon l'une quelconque des revendications précédentes, dans lequel l'agent oxydant inclut un acide gras oméga 3, un acide gras oméga 6, un ester de ceux-ci, ou un mélange de ceux-ci.

12. Article absorbant selon l'une quelconque des revendications précédentes, dans lequel l'agent oxydant inclut l'acide linoléique, l'acide α-linolénique, l'acide eicosadiénoïque, l'acide eicosatriénoïque, l'acide arachidonique, l'acide eicosapentanoïque, l'acide docosahexanoïque, un ester de ceux-ci, ou un mélange de ceux-ci.

13. Article absorbant selon l'une quelconque des revendications précédentes, dans lequel la composition décolorante comprend en outre un agent de lyse cellulaire.

14. Article absorbant selon l'une quelconque des revendications 1 à 13, dans lequel la couche perméable aux liquides est une feuille supérieure de l'article.

15. Article absorbant selon l'une quelconque des revendications 1 à 13, dans lequel la couche perméable aux liquides est une couche d'absorption, une couche de retard de transfert, ou une combinaison de celles-ci.
